# FASCICULE DE BREVET EUROPEEN

(11) **EP 0 582 698 B1**
(45) Date de publication et mention de la délivrance du brevet: **29.11.1995**
(21) Numéro de dépôt: 93904157.0
(22) Date de dépôt: 04.02.1993
(51) Int. Cl.: A61B 1/12, A61M 1/34, A61B 6/00

(54) **INSTALLATION POUR ANGIOSCOPIE**
VORRICHTUNG ZUR ANGIOSKOPIE
ANGIOSCOPY APPARATUS

(30) Priorité: 05.02.1992 FR 9201528
(43) Date de publication de la demande: 16.02.1994
(73) Titulaire: ROQUES, Francois, F-33400 Talence (FR)
(72) Inventeur: ROQUES, Francois, F-33400 Talence (FR)
(74) Mandataire: Dupuis, François
(86) Numéro de dépôt international: FR9300117
(87) Numéro de publication internationale: WO9315649

(56) Documents cités:
- EP-A- 0 165 519
- EP-A- 0 223 126
- FR-A- 2 397 197
- FR-A- 2 398 507
- GB-A- 2 009 863
- US-A- 3 900 022
- US-A- 5 053 002

## Description

Généralement, l'angioscopie est une méthode de contrôle en fin de procédure d'un geste pratique, le plus souvent sous contrôle radiologique. On peut citer par exemple, l'angioplastie transluminale, la pose d'endoprothèses, l'angioplastie laser...

L'angioscope est un instrument médical qui permet de visualiser l'intérieur des éléments du système cardio-vasculaire. En vue d'obtenir une image claire de la surface interne des vaisseaux, le sang doit être repoussé dans la région située à proximité de l'extrémité distale de l'angioscope. Le plus souvent, ce déplacement de sang s'effectue par irrigation d'une solution claire.

Une procédure d'angioscopie intervient rarement pour les procédures thérapeutiques. En effet, la plupart des méthodes thérapeutiques endovasculaires, telles que les atherectomies, les angioplasties transluminales, sont relativement longues. Une visualisation permanente du geste pratiqué nécessite des perfusions trop importantes de soluté translucide.

En dehors de situation opératoire particulière avec exclusion des segments d'amont et d'aval par clampage, les angioscopies s'effectuent le plus souvent, notamment lorsqu'elles sont pratiquées par ponction percutannée de l'artère ou de la veine, dans un système vasculaire ouvert. Dans ces conditions, il peut être nécessaire d'injecter une grande quantité de liquide pour obtenir une visualisation durable notamment dans le système artériel périphérique.

Par ailleurs, la clarification d'une artère périphérique nécessite, pour chasser le sang, des débits de solutés translucides très importants, pouvant atteindre 500 ml/mn. Or, si la perfusion rapide de soluté dépasse un litre, il en résulte une surchage hydrique brutale qui peut s'avérer dangereuse pour le patient, avec des risques d'oedème pulmonaire et de défaillance cardiaque.
La surchage hydrique constitue une restriction essentielle à une utilisation plus large de l'angioscopie.

Le cheminement de l'angioscopie jusqu'à la zone à visualiser s effectue sous contrôle radiologique. La visualisation des lésions se fait rapidement. Si un traitement est nécessaire, il est effectué sous contrôle radiologique. Les possibilités thérapeutiques dans un aussi court laps de temps, sont en fait très astreintes.

A ce jour, les sytèmes d'angioscopie comprennent des pompes d'irrigation plus ou moins sophistiquées.

Leurs principes consistent, à partir d'un réservoir de liquide translucide et au moyen d'une pompe le plus souvent, à amener le liquide en fort débit dans une artère, dans la région ou se situe l'extrémité de l'angioscope. Le liquide est le plus souvent irrigué par l'intermédiaire de l'angioscope. De nombreux systèmes d'angioscopie ont été proposé dont notamment celui défini dans le brevet US 5053002.

Ce brevet US enseigne un système permettant d'optimiser et de minimiser les quantités de liquides perfusés en donnant notamment la possibilité de pré-sélectionner ou de sélectionner deux débits d'irrigation variables.

On connaît également les techniques d'ultra filtration qui consistent à retirer un certain volume d'eau à partir du sang d'un patient. De telles techniques sont utilisées notamment chez les dialysés. Le principe général de ces techniques d'ultra filtration consiste à envoyer le sang sur une membrane semi-perméable autorisant le passage d'eau plasmatique ou ultrafiltrat. Cet ultrafiltrat contient l'eau, les électrolytes et certains éléments du sang dont le poids moléculaire est faible.

Le débit d'ultrafiltration dépend de la pression transmise sur la membrane, d'où l'intérêt de pouvoir utiliser une pompe d'un débit important. Il en résulte, dans des conditions optimales et en utilisant une membrane ayant de bonnes caractéristiques, qu'il est possible de récupérer un volume d'ultrafiltrat de dix litres par heure.

Ainsi, en cas d'insuffisance rénale aiguë ou chronique, primitive ou secondaire, il est partois nécessaire d'épurer un certain nombre de produits toxiques dans le sang et normalement transmis dans les urines.

L'ultrafiltration permet d'épurer ces produits lorsqu'ils sont de poids moléculaire faible ou moyen. De manière à retirer une grande quantité de ces produits "toxiques", il est nécessaire d'ultrafiltrer une très grande quantité d'eau plasmatique. Dans ces conditions, il est également nécessaire de restituer toute ou partie de cette eau plasmatique au patient. Le système restituant un certain volume hydrique au patient, est habituellement appelé circuit de substitution.

Enfin, dans le cadre des insuffisances rénales chroniques chez les dialysés, l'ultrafiltration est partois associée au décours de la dialyse entrant dans le cadre de l'hémodiafiltration. Dans ces conditions, sont placés de l'autre côté de la membrane, des bains de dialyse susceptibles d'augmenter le volume des échanges transmembranaires.

La technique d'ultrafiltration est également utilisée dans d'autres situations. Elle est notamment utilisée dans des techniques nouvelles de récupérations de sang. Son utilisation entre dans le cadre des pertes sanguines réalisées à l'occasion d'une intervention chirurgicale. Le sang sorti du lit vasculaire, recueilli dans les cavités naturelles comme la plèvre ou le péricarde par exemple, est recueilli dans un système qui, après avoir traité, lavé et filtré les éléments figurés du sang, fait passer ceux-ci au niveau d'une membrane d'ultrafiltration de manière à séparer les éléments figurés du sang et à les restituer au patient.

A ce jour, il n'a pas été proposé d'appliquer l'ultrafiltration pour faire face à une situation d'hyperhydratation avec augmentation de la masse sanguine et hémodilution iatrogène, comme c'est le cas par perfusion de liquide translucide au décours d'une procédure d'angioscopie. L'ultra-filtration est, dans les autres applications, associée à un appareillage et à une spécificité totalement différente de celle envisagée, de sorte que l'enseignement des techniques antérieures d'ultra-filtration, en suggère pas de manière évidente, la possibilité d'appliquer une telle technique dans le cas d'une procédure d'angioscopie.

Compte-tenu de cet état de la technique et des problèmes rencontrés avec les méthodes et installations précitées, le problème que se propose de résoudre l'invention est de réaliser en toute sécurité des angioscopies de longue durée qui permettent de contrôler le risque de surcharge hydrique, le cheminement de l'angioscope, l'examen des lésions et, éventuellement, un geste thérapeutique qui s'effectuerait sous contrôle angioscopique et non plus radiologique.

Le but de l'invention est de proposer une méthode sûre et fiable de contrôle des quantités de liquide translucide irrigué, l'opérateur pouvant alors s'attacher directement aux tâches diagnostiques ou thérapeutiques qu'il désire pratiquer. Enfin, un autre but est de simplifier l'ensemble de la procédure pour l'opérateur.

Pour résoudre ces différents problèmes il a été conçu selon l'invention, une installation conformément à la revendication 1.

Plus particulièrement, l'installation combine une pompe d'irrigation, un système d'extraction d'eau plasmatique fondé sur l'ultra-filtration et une unité de contrôle gérant le fonctionnement de la pompe et du système d'extraction, pour délivrer à l'opérateur, de manière simple, l'ensemble des informations nécessaires à la poursuite, en toute quiétude, de l'angioscopie.

Il apparait donc que l'installation selon l'invention résulte de la combinaison de deux systèmes connus séparemment, à savoir, l'angioscopie avec pompe d'irrigation, d'une part, et l'ultrafiltration, d'autre part, mais dont la combinaison procure un effet de synergie permettant de prolonger d'une manière durable la procédure d'angioscopie et d'augmenter l'efficacité de la méthode.

Cette solution permet de retirer du sang, 100 ml/mn de liquide, alors que pendant l'irrigation, on injecte une quantité toujours inférieure à 400 ml/mn. Ainsi, on peut envisager même en cas d'irrigation à très fort débit, une utilisation discontinue et tres prolongée de la pompe d'irrigation.

Le temps d'angioscopie, qui actuellement, est de deux à trois mn peut être porté à une vingtaine de minutes, voir beaucoup plus, sans danger pour le patient, puisque une quantité semblable d'eau plasmatique est retirée sur la même période.

Selon l'invention, à la différence des systèmes de pompe pour irrigation angioscopique, tels que précédemment décrits, l'élément essentiel n'est plus la quantité totale des liquides irrigués. En effet, seul compte la différence entre la quantité de liquide irrigué et celle récupérée par ultra-filtration.

Enfin, dans le cadre d'angioscopie particulièrement longue, doivent également être connues et comptabilisées les urines, mais aussi les pertes en liquide d'irrigation plus ou moins mêlées au sang, qui peuvent se voir dans le champ opératoire.

Dans une forme de réalisation préférée, l'installation comprend :
- à la sortie d'un réservoir contenant un liquide d'irrigation, un moyen apte à envoyer ledit liquide dans un vaisseau sanguin que l'on désire explorer, par un appareil du type angioscope, le liquide translucide étant injecté le plus souvent par l'angioscope dans le vaisseau sanguin.
- un moyen apte à retenir le sang du patient pour l'envoyer dans un hémofiltre relié, d'une part, à un réservoir d'ultrafiltration et, d'autre part, à un circuit de restitution de sang ultrafiltré éventuellement équipé d'un réservoir dont la sortie est équipée pour restituer le sang ainsi traité au patient.

Avantageusement, les moyens sont constitués par des pompes à débits variables.

A la sortie de la pompe et en amont de l'angioscope, est placé un piège à bulles, un détecteur de bulles pouvant également être implanté. A la sortie du réservoir du sang ultrafiltré, est montée une pompe pour restituer le sang au patient.

Pour résoudre le problème posé de recueillir et de restituer le sang dans les conditions indiquées, on peut envisager les solutions suivantes :
- Le sang recueilli pour ultrafiltration et celui ultrafiltré restitué au patient, peuvent l'être par une seule ponction, dans une voie veineuse centrale, au moyen d'un catheter à double voie.
- Le sang recueilli pour ultrafiltration et celui ultrafiltré restitué au patient, peuvent l'être par deux points de ponction différents au moyen de deux catheters à une voie.

Pour résoudre le problème posé d'assurer un fonctionnement dans les meilleurs conditions de l'installation, les différentes pompes sont asservies par un système informatique de contrôle, apte à assurer un fonctionnement simultané en mettant en permanence, en comparaison la quantité d'eau injectée au patient et celle recueillie dans le réservoir d'ultrafiltration. Peuvent être aussi considérés, le volume de sang ultrafiltré stocké dans une poche tampon, qui représente le réservoir du sang ultrafiltré, et auquel est asservi le débit de la pompe, mais aussi l'éventuel volume d'urine stocké et pesé dans une poche à urine.

Plus particulièrement, le fonctionnement de ce système informatique ou unité de contrôle, peut être indiqué comme suit :
- MESURE 1 : volume de soluté translucide injecté.
   Cette mesure peut être effectuée soit par pesée de la poche réservoir du liquide d'irrigation, soit plus particulièrement par l'intermédiaire d'un compteur placé sur la pompe d'irrigation et permettant de connaître en permanence le débit d'irrigation et le volume total de liquide translucide irrigué.
- MESURE 2 : volume d'ultra-filtrat situé dans la poche réservoir d'ultrafiltration, la méthode de mesure la plus simple étant probablement un système de pesée.
- MESURE 3 : lorsque le retour du sang ultrafiltré est doté d'une poche tampon, celle-ci peut également faire l'objet d'une mesure par pesée. Dans le but de simplifier le montage et dans la conformation type du système, la poche tampon ou la poche de petit volume n'est pas utilisée, de manière à pouvoir négliger le volume total stocké.
- MESURES 4 ET 5 : compte-tenu des possibilités d'angioscopie longue, le système de contrôle des volumes doit également intégrer le volume total des urines et le volume éventuel de perte des liquides d'irrigation plus ou moins mêlés au sang d'un champ opératoire, dans le cas où le foyer opératoire ne serait pas parfaitement étanche aux liquides.

Ces données doivent pouvoir être entrées manuellement au niveau de la console de la double pompe qui doit être dotée d'un clavier de saisie. En intégrant l'ensemble de ces données : volume total perfusé, volume total de l'ultra-filtrat et éventuellement volume de la poche tampon, volume d'urine, volume des liquides perdus dans un champ opératoire, l'unité centrale de contrôle calcule la valeur différentielle des entrées et des sorties (DV).

Par ailleurs, l'unité centrale a pour but de gérer la sécurité de la procédure d'angioscopie, en ayant pour mission d'assurer une sécurité absolue. Dans ce but, l'unité centrale bloque le système des pompes de manière sélective sur l'une ou l'autre des pompes, lorsqu'une situation dangereuse est susceptible d'apparaitre, à savoir, dans l'hypothèse de conditions physiologiques à définir : DV < -500 ml, DV > 1 litre, variation de DV trop brutale (DV> 800 ml en 2 mn par exemple).

Enfin, il doit exister un système de communication simple entre l'opérateur et l'unité centrale de contrôle. Ce système de communication doit permettre de simplifier la tâche de l'opérateur.

L'invention est exposée, ci-après plus en détail à l'aide des dessins annexés dans lesquels :

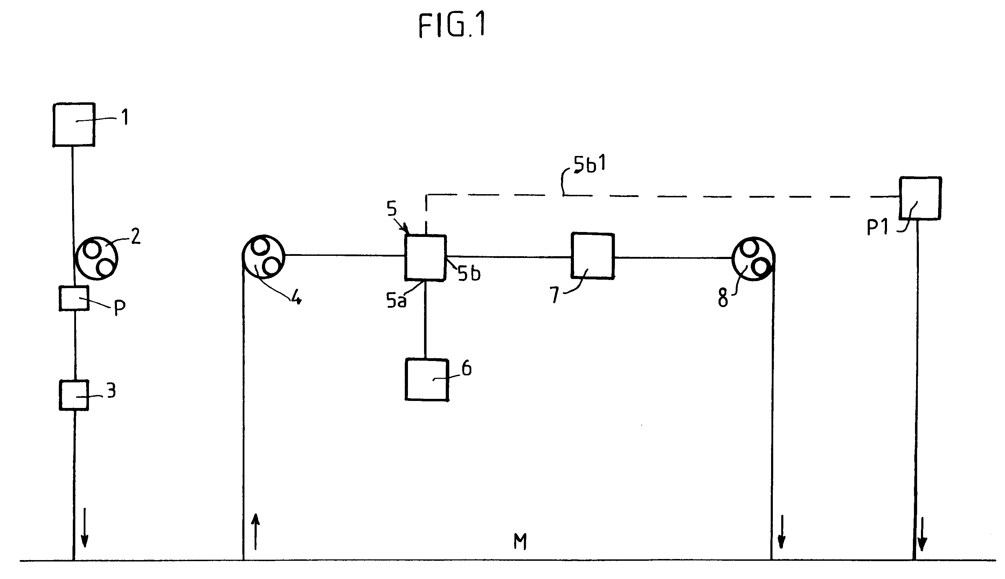

La figure 1 est une vue montre le principe d'irrigaiton et d'ultrafiltration pour angioscopie selon l'invention.
La figure 2 est une vue à caractère purement schématique, montrant un exemple de réalisation de l'installation d'angioscopie selon l'invention.

Cette installation comprend à la sortie d'un réservoir (1) contenant le liquide d'irrigation, une pompe (2) reliée à l'angioscope (3). Le liquide d'irrigation est injecté dans la masse sanguine (M) du patient par l'intermédiaire de l'angioscope (3) ou directement dans le vaisseau que l'on veut explorer avec l'angioscope. Un piège à bulles (P) peut éventuellement être monté à la sortie de la pompe (2). A noter que la pompe envoie un liquide physiologique, translucide, tel que celui utilisé pour la substitution lors d'hémofiltration à environ 37°C.

Une seconde pompe (4) recueille le sang du patient à ultrafiltrer. Cette pompe (4) dirige le sang sur un appareil hémofiltre (5). Cet hémofiltre (5) présente deux sorties (5a) (5b). La sortie (5a) est reliée à un réservoir d'ultrafiltrat (6), tandis que la sortie (5b) est reliée à un réservoir de sang ultrafiltré (7).

A la sortie du réservoir (7), une pompe (8) récupère le sang pour le restituer dans la masse sanguine du patient.

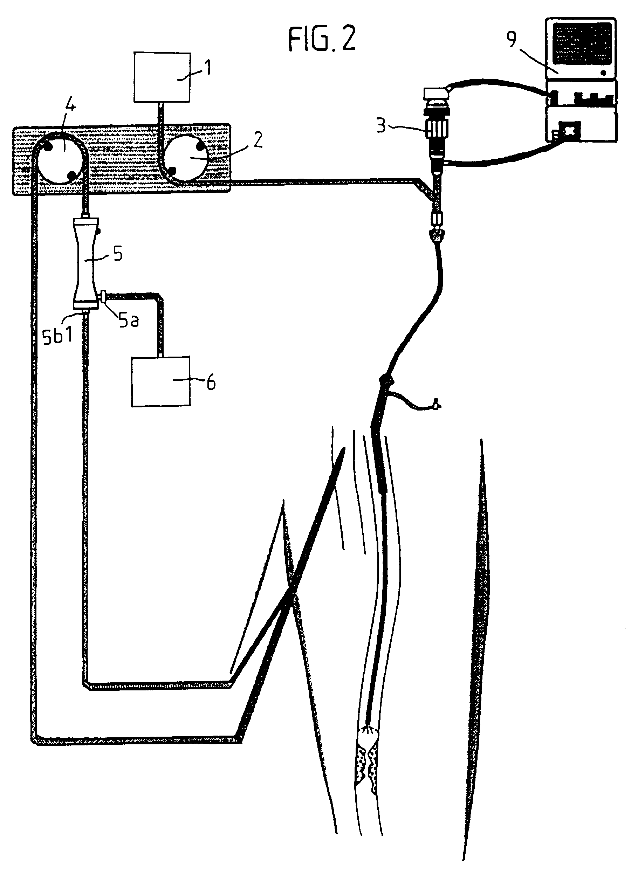

Dans un but de simplification et dans une forme de réalisation préférée, telle qu'illustrée à la figure 2, à la sortie de l'hémofiltre (5), le sang peut être restitué au malade, directement, c'est-à-dire sans l'utilisation de la pompe (8). Cette voie de restitution du sang (5b1) est alors simplement dotée d'un piège à bulles (P1).

Les différentes pompes (2) et (4) notamment, sont asservies par un système informatique de contrôle, apte à assurer un fonctionnement simultané en mettant en permanence, en comparaison, la quantité d'eau injectée au patient et celle recueillie dans le réservoir d'ultrafiltrat (6) et la quantité de sang ultra filtrée stockée dans la poche tampon (7). On peut également prendre en compte, la quantité d'urine produite et pesée pendant l'angioscopie et éventuellement les pertes liquidiennes dans le champs opératoire.

On rappelle, comme indiqué dans le préambule, que ce système informatique (9) permet, d'une part, d'analyser un certain nombre de mesures, notamment le volume de soluté translucide injecté, le volume d'ultra-filtrat, la pesée de la poche tampon lorsqu'elle existe, l'intégration du volume total des urines et du volume éventuel de pertes des liquides d'irrigation et, d'autre part, de gérer la sécurité de la procédure.

A noter également qu'un certain nombre de constantes doivent pouvoir être sélectionnées par l'opérateur, tandis que des ordres directs doivent pouvoir être adressés à l'unité de contrôle. Il est prévu à cet effet un clavier sur la console de la double pompe, permettant par exemple à un assistant qui n'est pas dans le champ opératoire, d'effectuer les réglages.

Les constantes sélectionnées sont la valeur de la vitesse rapide d'irrigation, la valeur de la vitesse lente d'irrigation, les entrées manuelles des quantités d'urine et des quantités de liquide perdu dans le champ opératoire. Un ordre d'arrêt des pompes peut être composé par l'intermédiaire de ce clavier. Un bouton doit également être prévu pour donner l'ordre à la pompe d'irrigation de fonctionner en vitesse rapide et un deuxième en vitesse lente.

Dans un souci de simplification des manipulations, il est prévu que l'ensemble de ces informations sont adressées à l'unité centrale par l'intermédiaire d'un boitier de télécommande. Ce boitier de télécommande est contenu dans une poche stérile qui pourrait être mise ainsi simplement à la disposition de l'opérateur.

En outre, de manière à ce que l'opérateur puisse s'occuper exclusivement de la manipulation de l'angioscope et du contrôle du geste pratiqué sur l'écran vidéo, il a été conçu que les informations concernant le fonctionnement de la double pompe puisse être portées dans le champ visuel direct de l'opérateur à savoir sur l'écran vidéo faisant partie intégrante du système de visualisation de l'angioscope. En effet, en angioscopie, compte-tenu des caractéristiques des fibres, seule une petite partie de l'image est utilisée au centre de l'écran pour visualiser l'artère.

Dans ces conditions, il existe sur le moniteur, au dessus ou en dessous de cette image, la possibilité de faire figurer les constantes de sécurité concernant le fonctionnement de la double pompe. Ce système pourrait permettre à l'opérateur de se concentrer exclusivement sur le geste qu'il pratique. Il est donc conçu un système permettant de raccorder la double pompe et le moniteur. Les informations importantes qui pourraient figurer sur l'écran du moniteur sont :
- la valeur instantanée du DV,
- la valeur du débit d'irrigation,
- le système étant susceptible de fonctionner de manière intermittente, pour assurer la sécurité du patient, en cas de blocage temporaire par l'unité centrale, il est nécessaire de faire figurer le délai avant la prochaine séquence et la durée prévisible de la prochaine séquence d'angioscopie compte-tenu du débit d'irrigation de la pompe pré-sélectionnée.

Le recueil et la restitution du sang dans les conditions indiquées, peut s'effectuer par tout moyen connu et approprié. Par exemple, on peut utiliser un catheter double voies placé dans une voie veineuse centrale, ou bien on peut utiliser deux catheters une voie placés dans deux voies veineuses différentes.

Les avantages ressortent bien de la description.

## Revendications

1. Installation pour angioscopie, caractérisée en ce qu'elle comprend en combinaison, un système d'angioscopie (1) (2) (3) avec irrigation à l'aide d'un liquide physiologique translucide et un système d'ultrafiltration (4) (5) (6) (7) apte à retirer un certain volume d'eau à partir du sang du patient pour tenir compte d'une hyper-hydration provoquée par ladite irrigation.

2. Installation selon la revendication 1, caractérisée en ce que l'installation combine une pompe d'irrigation (2), un système (4) d'extraction d'eau plasmatique fondé sur l'ultra-filtration et une unité de contrôle (9) gérant le fonctionnement de la pompe et du système d'extraction.

3. Installation selon la revendication 2, caractérisée en ce qu'elle comprend :
- à la sortie d'un réservoir (1) contenant un liquide d'irrigation, la pompe (2) apte à envoyer ledit liquide translucide dans un vaisseau sanguin que l'on désire visualiser par angioscopie, le plus souvent au travers d'un appareil du type angioscope (3).
- une pompe (4), un hémofiltre (5) et un réservoir d'ultrafiltration (6), la pompe étant (4) apte à recueillir le sang du patient pour l'envoyer dans l'hémofiltre (5) dont l'une des sorties (5a) est reliée au réservoir d'ultrafiltration (6) et, dont l'autre sortie (5b1) est reliée directement au malade.

4. Installation selon la revendication 2, caractérisée en ce qu'elle comprend :
- à la sortie d'un réservoir (1) contenant un liquide d'irrigation, la pompe (2) apte à envoyer ledit liquide translucide dans un vaisseau sanguin que l'on désire visualiser par angioscopie, le plus souvent au travers d'un appareil du type angioscope (3)
- une pompe (4), un hémofiltre (5) et un réservoir d'ultrafiltration (6) la pompe (4) étant apte à recueillir le sang du patient pour l'envoyer dans l'hémofiltre (5) dont l'une des sorties (5a) est reliée au réservoir d'ultrafiltration (6) et, dont l'autre sortie (5b) est reliée à un réservoir de sang ultrafiltré (7) dont la sortie est équipée pour restituer le sang ainsi traité au patient.

5. Installation selon l'une quelconque des revendications 3 et 4, caractérisée en ce qu'à la sortie de la pompe (2) montée en amont de l'angioscope (3), est placé un piège à bulles.

6. Installation selon l'une quelconque des revendications 3 et 4, caractérisée en ce qu'à la sortie du réservoir du sang ultrafiltré (7), est montée une pompe (8) pour restituer le sang au patient.

7. Installation selon l'une quelconque des revendications 3 et 4, caractérisée en ce que l'hémofiltre (5) présente une sortie (5b1) pour restituer le sang ultrafiltré, directement au patient, un piège à bulles (P1) étant monté sur cette sortie (5b1).

8. Installation selon l'une quelconque des revendications 3 et 4, caractérisée en ce qu'à la sortie du réservoir du sang ultrafiltré (7) et à l'entrée de la pompe (4) pour le prélèvement du sang, est monté un catheter double voies placé dans une voie veineuse centrale.

9. Installation selon l'une quelconque des revendications 3 et 4, caractérisée en ce qu'à la sortie du réservoir du sang ultrafiltré (7) et à l'entrée de la pompe (4) pour le prélèvement du sang, sont montés deux catheters une voie placés dans deux voies veineuses différentes.

10. Installation selon l'une quelconque des revendications 1 à 9, caractérisée en ce que les différentes pompes (2) (4) (8) sont asservies par un système informatique apte à assurer un fonctionnement simultané en mettant en permanence, en comparaison la quantité d'eau injectée au patient et celle recueillie dans le réservoir d'ultrafiltration (6) et la quantité de sang ultra filtrée stockée dans la poche tampon (7).

## Claims

1. A set-up for angioscopy characterized in that it consists of a system of angioscopy (1) (2) (3) with irrigation by means of translucent saline, coupled with a system of ultrafiltration (4) (5) (6) (7) capable of removing a certain volume of water from the patient's blood, to compensate for hyperhydration caused by said irrigation.

2. A set-up according to claim 1, characterized in that said set-up includes an irrigation pump (2), a system of plasma water extraction (4) based on ultrafiltration principles and a control unit (9) managing the operation of said pump and of said extraction system.

3. A set-up according to claim 2, characterized in that it includes:
- at the outlet of a tank (1) containing an irrigation fluid, a pump (2) capable of pulsing said translucid liquid into a blood vessel which one wishes to visualize by angioscopy, usually by means of an angioscope-like instrument (3),
- a pump (4), a haemofilter (5) and an ultrafiltration tank (6), said pump (4) being capable of drawing the patient's blood and pulsing it to said haemofilter (5) one outlet of which (5a) is linked to said ultrafiltration tank (6), the other outlet (5b1) being linked directly to the patient's blood vessel.

4. A set-up according to claim 2, characterized in that it includes:
- at the outlet of a tank (1) containing an irrigation fluid, a pump (2) capable of pumping said translucent liquid into a blood vessel which one wishes to visualize by angioscopy, usually by means of an angioscope-like instrument (3).
- a pump (4), a haemofilter (5) and an ultrafiltration tank (6), said pump (4) being capable of drawing the patient's blood and pulsing it to said haemofilter (5), one outlet of which (5a) is linked to said ultrafiltration tank (6), and the other outlet (5b) being linked to an ultrafiltration blood tank (7) the outlet of which is equipped with a line for returning blood to the patient.

5. A set-up according to either of claims 3 or 4, characterized in that a bubble-trap is placed at the outlet of said pump (2) upstream of the angioscope (3).

6. A set-up according to either of claims 3 or 4, characterized in that a pump (8) for returning blood to the patient is placed at the outlet of said ultrafiltered blood tank (7).

7. A set-up according to either of claims 3 or 4, characterized in that said haemofilter (5) is provided with an outlet (5b1) for returning ultrafiltered blood directly to patient, said outlet (5b1) being equipped with a bubble-trap (P1).

8. A set-up according to either of claims 3 or 4, characterized in that at the outlet of said ultrafiltered blood tank (7) and at the inlet of said pump (4) for blood collection, a two-line catheter is mounted and inserted into a vein of the central venous system.

9. A set-up according to either of claims 3 or 4, characterized in that at the outlet of said ultrafiltered blood tank (7) and at the inlet of said pump (4) for blood collection, two single-line catheters are mounted and inserted into two different venous pathways.

10. A set-up according to any of claims 1 to 9, characterized in that said pumps (2) (4) (8) are controlled by a monitoring system capable of ensuring their simultaneous operation and continuously displaying comparison data between the amounts of water injected to the patient and those received by said ultrafiltration tank (6) and the amounts of ultrafiltered blood stored in the buffer bag (7).

## Patentansprüche

1. Angioskopieanlage bestehend aus einem Angioskopiesystem (1) (2) (3) mit Irrigation durch eine transluzide physiologische Flüssigkeit und einem Ultrafiltrationssystem (4) (5) (6) (7), das geeignet ist, dem Blut des Patienten ein bestimmtes Wasservolumen zu entziehen, um eine durch die Irrigation hervorgerufene Hyperhydratation auszugleichen.

2. Anlage nach Anspruch 1, dadurch gekennzeichnet, daß eine Irrigationspumpe (2) mit einem auf der Ultrafiltration beruhenden System (4) zur Abscheidung von Wasser aus dem Plasma und einem Kontrollgerät (9) für die Funktionsüberwachung der Pumpe und des Wasserextraktionssystems kombiniert ist.

3. Anlage nach Anspruch 2, dadurch gekennzeichnet, daß sie folgende Teile umfaßt:
- am Ausgang eines Behälters (1) mit einer Irrigationsflüssigkeit die Pumpe (2), die geeignet ist, die transluzide Flüssigkeit in ein Blutgefäß zu pumpen, das durch Angioskopie, meistens durch ein angioskopartiges Gerät (3), sichtbar gemacht werden soll;
- eine Pumpe (4), einen Blutfilter (5) und einen Ultrafiltrationsbehälter (6), wobei die Pumpe (4) geeignet ist, das Blut des Patienten aufzunehmen, um es in den Blutfilter (5) zu befördern, von dem ein Ausgang (5a) mit dem Ultrafiltrationsbehälter (6) und der andere Ausgang (5b1) direkt mit dem Kranken in Verbindung steht.

4. Anlage nach Anspruch 2, dadurch gekennzeichnet, daß sie folgende Teile umfaßt:
- am Ausgang eines Behälters (1) mit einer Irrigationsflüssigkeit die Pumpe (2), die geeignet ist, die transluzide Flüssigkeit in ein Blutgefäß zu pumpen, das durch Angioskopie, meistens durch ein angioskopartiges Gerät (3), sichtbar gemacht werden soll;
- eine Pumpe (4), einen Blutfilter (5) und einen Ultrafiltrationsbehälter (6), wobei die Pumpe (4) geeignet ist, das Blut des Patienten aufzunehmen, um es in den Blutfilter (5) zu befördern, von dem ein Ausgang (5a) mit dem Ultrafiltrationsbehälter (6) und der andere Ausgang (5b) mit einem Behälter für das ultrafiltrierte Blut (7) in Verbindung steht, dessen Ausgang entsprechend ausgestattet ist, um das derart behandelte Blut an den Patienten zurückzugeben.

5. Anlage nach einem der Ansprüche 3 und 4, dadurch gekennzeichnet, daß am Ausgang der Pumpe (2) vor dem Angioskopen (3) ein Blasenfänger angeordnet ist.

6. Anlage nach einem der Ansprüche 3 und 4, dadurch gekennzeichnet, daß am Ausgang des Behälters mit dem ultrafiltrierten Blut (7) eine Pumpe angebracht ist, um das Blut an den Patienten zurückzugeben.

7. Anlage nach einem der Ansprüche 3 und 4, dadurch gekennzeichnet, daß der Blutfilter (5) einen Ausgang (5b1) aufweist, um das ultrafiltrierte Blut direkt an den Patienten zurückzugeben, wobei an diesem Ausgang (5b1) ein Blasenfänger (P1) angebracht ist.

8. Anlage nach einem der Ansprüche 3 und 4, dadurch gekennzeichnet, daß am Ausgang des Behälters mit dem ultrafiltrierten Blut (7) und am Eingang der Pumpe (4) für die Blutentnahme ein Doppelkanalkatheter in einer zentralen Venenbahn angebracht ist.

9. Anlage nach einem der Ansprüche 3 und 4, dadurch gekennzeichnet, daß am Ausgang des Behälters mit dem ultrafiltrierten Blut (7) und am Eingang der Pumpe (4) für die Blutentnahme zwei Einkanalkatheter in zwei unterschiedlichen Venenbahnen angebracht sind.

10. Anlage nach einem der Ansprüche 1-9, dadurch gekennzeichnet, daß die einzelnen Pumpen (2) (4) (8) durch ein EDV-System geregelt werden, das geeignet ist, deren gleichzeitigen Betrieb zu gewährleisten, wobei die dem Patienten eingeflößte Wassermenge, die in dem Ultrafiltrationsbehälter (6) gesammelte Wassermenge und die in dem Pufferbehälter (7) gespeicherte ultrafiltrierte Blutmenge laufend verglichen werden.
